Europäisches Patentamt

European Patent Office

Office européen des brevets

(11) Numéro de publication: **0 418 110 A1**

# DEMANDE DE BREVET EUROPEEN

(21) Numéro de dépôt: 90402382.7

(22) Date de dépôt: 29.08.90

(51) Int. Cl.⁵: **A61K 31/195**, A61K 31/725, A61K 37/64

(30) Priorité: 30.08.89 FR 8911379

(43) Date de publication de la demande:
20.03.91 Bulletin 91/12

(84) Etats contractants désignés:
GR

(71) Demandeur: Chevance, Léon Georges
1, Avenue Jean Jaurès
FR-94340 Joinville Le Pont(FR)

(72) Inventeur: Chevance, Léon Georges
1, Avenue Jean Jaurès
FR-94340 Joinville Le Pont(FR)

(74) Mandataire: Clisci, Serge et al
S.A. FEDIT-LORIOT & AUTRES 38, avenue Hoche
F-75008 Paris(FR)

(54) Nouvelle utilisation des anticompléments pour le traitement des parodontoses.

(57) On utilise pour le traitement par voie locale dans la bouche des manifestations pathologiques gingivo-dentaires précurseurs de la parodontose, des médicaments renfermant une proportion active d'un inhibiteur du complément soluble dans la salive et non agressif pour la muqueuse gingivale; notamment un mélange de gomme arabique et de L-glutamate de sodium.

## NOUVELLE UTILISATION DES ANTICOMPLEMENTS POUR LE TRAITEMENT DES PARODONTOSES

La présente invention concerne l'industrie pharmaceutique. Elle a pour objet une nouvelle utilisation d'une substance choisie parmi les anticompléments solubles dans la salive et non agressifs vis-à-vis de la muqueuse gingivale, pour l'obtention d'un médicament destiné à une utilisation en thérapeutique humaine et vétérinaire en tant qu'agent de traitement, par voie locale dans la bouche, des manifestations pathologiques gingivo-dentaires précurseurs de la parodontose.

L'invention a également pour objet des médicaments pour le traitement local de manifestations pathologiques gingivo-dentaires précurseurs de la parodontose, caractérisés en ce qu'ils renferment une proportion active d'au moins un inhibiteur du complément soluble dans la salive et non agressif pour la muqueuse gingivale.

Dans un mode de réalisation avantageux, l'inhibiteur du complément fait partie du groupe comprenant l'acide L-glutamique, le L-glutamate de sodium, les sels de magnésium et de sodium de l'acide N-acétylaspartique glutamique, la gomme arabique et leurs associations.

Il est pratique que l'inhibiteur du complément soit mélangé à une gomme a mâcher.

Suivant un mode de réalisation préféré, un médicament suivant l'invention renferme un mélange de gomme arabique et d'un autre inhibiteur du complément, il peut être avantageusement conditionné en tablettes à sucer d'environ 0,5 à 1,5 grammes, renfermant environ 92 à 96 parties pondérales de gomme arabique et environ 4 à 8 parties pondérales de sel monosodique de l'acide L-glutamique.

Un médicament suivant l'invention peut renfermer, en outre, au moins une substance édulcorante et/ou aromatisante, et/ou au moins une autre substance douée d'une activité thérapeutique, notamment antibiotique.

### Exemple

Préparation : On mélange intimement 100 grammes de gomme arabique réduite en poudre fine avec 6 grammes de L glutamate monosodique. On empâte à température ambiante normale avec de l'eau distillée en malaxant pour obtenir une pâte molle homogène. On évapore l'eau par simple exposition à l'air, ou sous vide, ou par chauffage à moins de 100°C, pour obtenir une galette solide d'environ 0,5 cm d'épaisseur, que l'on découpe en tablettes carrées d'environ 1 cm de côté.

Aucun additif conservateur n'est généralement nécessaire, pas plus qu'un édulcorant ou un arôme, le goût des tablettes évoquant celui d'un bouillon de viande. Toutefois, il est possible d'ajouter à volonté du sucre ou, de préférence, un édulcorant de synthèse ainsi qu'un parfum alimentaire.

Traitement : On prescrit à un patient, souffrant d'inflammations douloureuses des gencives et de petits saignements provoqués par le brossage des dents, de sucer des fois par jour, après les soins d'hygiène dentaire indispensables, des tablettes (soit quatre par jour) que l'on laisse fondre lentement dans la bouche, de préférence au contact des parties douloureuses.

Au bout de deux semaines de traitement, on constate l'arrêt des saignements et une diminution marquée des douleurs gingivales. On poursuit le traitement jusqu'à disparition complète des manifestations pathologiques, ce qui peut demander plusieurs mois à un an suivant le stade d'évolution et la gravité des lésions initiales. Le traitement prolongé ne présente aucun inconvénient ni contre-indication.

La présente invention concerne aussi un procédé de préparation d'une composition thérapeutique humaine ou vétérinaire vis-à-vis des manifestations-pathologiques gingivo-dentaires précuseurs de la parodontose, ledit procédé étant caractérisé en ce qu'on fait appel à un moyen anticomplément soluble dans la salive et non agressif vis-à-vis de la muqueuse gingivale.

### Revendications

1. Utilisation d'une substance choisie parmi l'ensemble constitué par les moyens anticompléments solubles dans la salive et non agressifs vis-à-vis de la muqueuse gingivale, pour l'obtention d'un médicament destiné à une utilisation en thérapeutique humaine et vétérinaire en tant qu'agent de traitement, par voie locale dans la bouche, des manifestations pathologiques gingivo-dentaires précurseurs de la parodontose.

2. Utilisation suivant la revendication 1, caractérisée en ce que le moyen anti-complément soluble dans la salive est choisi parmi l'ensemble constitué par l'acide L-glutamique, le L-glutamate de sodium, le sel de sodium de l'acide N-acétylaspartyl-glutamique, le sel de magnésium de l'acide N-acétylaspartyl-glutamique, la gomme arabique et leurs mélanges.

3. Utilisation suivant la revendication 2, caractérisée en ce que le L-glutamate de sodium est le L-glutamate monosodique.

4. Utilisation suivant la revendication 2, caractérisée en ce que le moyen anti-complément est un mélange synergique de gomme arabique et d'un autre inhibiteur du complément.

5. Médicament pour le traitement local de manifestations, pathologiques gingivo-dentaires précurseurs de la parodontose, caractérisé en ce qu'il renferme une proportion active d'au moins un moyen anti-complément qui inhibe le complément, est soluble dans la salive et est non-agressif pour la muqueuse gingivale.

6. Médicament suivant la revendication 5, caractérisé en ce que le moyen anti-complément inhibant le complément est choisi parmi l'ensemble comprenant l'acide L-glutamique, le L-glutamate de sodium, le sel de magnésium de l'acide N-acétylaspartyl-glutamique, le sel de sodium de l'acide N-acétylaspartyl-glutamique, la gomme arabique et leurs mélanges.

7. Médicament suivant l'une quelconque des revendications 5 et 6, caractérisé en ce qu'il renferme un mélange d'inhibiteur du complément et de gomme à mâcher.

8. Médicament suivant l'une quelconque des revendications 5 à 7, caractérisé en ce qu'il renferme un mélange synergique de gomme arabique et d'un autre inhibiteur du complément.

9. Médicament suivant la revendication 8, caractérisé en ce qu'il est conditionné sous forme de tablettes à sucer d'environ 0,5 à 1,5 grammes, renfermant chacune environ 92 à 96 parties en poids de gomme arabique et environ 4 à 8 parties en poids de L-glutamate monosodique.

10. Procédé de préparation d'une composition thérapeutique humaine ou vétérinaire vis-à-vis des manifestations pathologiques gingivo-dentaires précuseurs de la parodontose, ledit procédé étant caractérisé en ce qu'on fait appel à un moyen anticomplément soluble dans la salive et non agressif vis-à-vis de la muqueuse gingivale.

| | | |
|---|---|---|
| Office européen<br>des brevets | **RAPPORT PARTIEL<br>DE RECHERCHE EUROPEENNE**<br>qui selon la règle 45 de la Convention sur le brevet<br>européen est considéré, aux fins de la procédure ultérieure,<br>comme le rapport de recherche européenne | Numéro de la demande<br><br>EP 90 40 2382 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | JOURNAL OF PHARMACEUTICAL SCIENCES, vol. 66, no. 10, octobre 1977, pages 1367-1377, Washington, US; B.J. JOHNSON: "Complement: A host defense mechanism ready for pharmacological manipulation?"<br><br>* Pages 1372,1374 * | <br><br><br><br><br>1,5,7,<br>10 | A 61 K 31/195<br>A 61 K 31/725<br>A 61 K 37/64 |
| Y | | 2-4,6,<br>8-9 | |
| | -- | | |
| Y | JOURNAL OF PERIODONTAL RESEARCH, vol. 17, no. 3, 1982, pages 257-262, Copenhague, DK; E.T. LALLY et al.: "Biosynthesis of complement components in chronically inflamed gingiva"<br><br>* Abrégé; Discussion * | <br><br><br><br><br>1-10 | DOMAINES TECHNIQUES RECHERCHES (Int. Cl.4)<br><br>A 61 K |
| | -- ./. | | |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 3,9

Revendications ayant fait l'objet de recherches incomplètes: 1-2,4-8,10

Revendications n'ayant pas fait l'objet de recherches:

Raison pour la limitation de la recherche:

Un composé n'est pas suffisamment caractérisé par son activité biologique (moyen anticomplément; rev. 1,5,10 ou inhibiteur du complément; rev. 4,7,8). Voir Art 84 CEB.

| Lieu de la recherche | Date d'achèvement de la recherche | Examinateur |
|---|---|---|
| LA HAYE | 10-12-1990 | DULLAART |

**Office européen des brevets**

# RAPPORT PARTIEL DE RECHERCHE EUROPEENNE

| | DOCUMENTS CONSIDERES COMME PERTINENTS | | CLASSEMENT DE LA DEMANDE (Int. Cl.4) |
|---|---|---|---|
| Catégorie | Citation du document avec indication, en cas de besoin, des parties pertinentes | Revendication concernée | |
| Y | IMMUNOPHARMACOLOGY, vol. 8, 1984, pages 27-35, Elsevier Science Publishers B.V., Amsterdam, NL; A. TAKADA et al.: "Inhibition of the activation of the first component of complement, C1, by various amino acids or peptides"<br><br>* Figures 4,7,8; Discussion * | 1-10 | |
| Y | KLINISCHE MONATSBLAETTER FUER AUGENHEILKUNDE, vol. 190, no. 4, 1987, pages 340-341, F. Enke Verlag, Stuttgart, DE; O.P. VAN BIJSTERVELD: "Conjonctivites allergiques subaiguës et chroniques"<br><br>* En entier * | 1-10 | **DOMAINES TECHNIQUES RECHERCHES (Int. Cl.4)** |
| X | JP-B-48 43 419 (OKUNU PHARMACEUTICAL INDS)<br><br>* Abrégé * | 4,8 | |
| A | JP-A-60 237 018 (TANPEI SEIYAKU K.K.)<br><br>* Abrégé * | 4,8,9 | |
| A | ARZNEIMITTELFORSCHUNG, vol. 31, no. 7, 1981, pages 1135-1140, Aulendorf, DE; G. PÖCH: "Quantitative Ermittlung potenzierender oder hemmender Kombinationswirkungen gleichsinnig wirkender Pharmaka"<br><br>* En entier * | 4,8,9 | |

OEB Form 1505.3 06.78